# EUROPÄISCHE PATENTSCHRIFT

(11) **EP 2 134 464 B1**
(45) Veröffentlichungstag und Bekanntmachung des Hinweises auf die Patenterteilung: **16.04.2014**
(21) Anmeldenummer: 08707312.8
(22) Anmeldetag: 25.01.2008
(51) Int. Cl.: B01J 20/20, B01J 20/28, B01J 20/30, C01B 31/08, C01B 31/10, A61L 9/014, B01D 53/02, A61K 33/44, C02F 1/28

(54) **HOCHLEISTUNGSADSORBENTIEN AUF DER BASIS VON AKTIVKOHLE MIT HOHER MESO- UND MAKROPOROSITÄT**
HIGH-PERFORMANCE ADSORBENTS BASED ON ACTIVATED CARBON HAVING HIGH MESO- AND MACROPOROSITY
PRODUIT ADSORBANT HAUTE PERFORMANCE À BASE DE CHARBON ACTIF AYANT UNE MÉSOPOROSITÉ ET UNE MACROPOROSITÉ ÉLEVÉES

(30) Priorität: 14.03.2007 DE 102007012963; 25.10.2007 DE 102007050971
(43) Veröffentlichungstag der Anmeldung: 23.12.2009
(73) Patentinhaber: Blücher GmbH, 40699 Erkrath (DE)
(72) Erfinder: BÖHRINGER, Bertram, 42115 Wuppertal (DE); FICHTNER, Sven, 14727 Premnitz (DE); GIEBELHAUSEN, Jean-Michael, 14712 Rathenow (DE)
(74) Vertreter: Von Rohr
(86) Internationale Anmeldenummer: PCT/EP2008/000606
(87) Internationale Veröffentlichungsnummer: WO 2008/110233

(56) Entgegenhaltungen:
- EP-A- 1 049 116
- EP-A- 1 666 649
- WO-A-2004/046033
- THIEME RÖMPP ONLINE: "Akivkohle" INTERNET CITATION, [Online] August 2004 (2004-08), XP002468792 Gefunden im Internet: URL:http://www.roempp.com/prod/index1.html > [gefunden am 2008-02-13]

## Beschreibung

Die vorliegende Erfindung betrifft das technische Gebiet der Adsorption. Insbesondere betrifft die vorliegende Erfindung Hochleistungsadsorbentien auf der Basis von Aktivkohle mit hoher Meso- und Makroporosität und ein Verfahren zu deren Herstellung sowie die Verwendung dieser Hochleistungsadsorbentien, insbesondere für Adsorptionsfiltermaterialien, für die Lebensmittelindustrie (z. B. zur Aufbereitung und/oder Entfärbung von Lebensmitteln), für die Adsorption von Giftstoffen, Schadstoffen und Gerüchen, insbesondere aus Gas- bzw. Luftströmen, zur Reinigung oder Aufbereitung von Gasen, wie insbesondere Luft, und von Flüssigkeiten, wie insbesondere Wasser, zur Anwendung in der Medizin bzw. Pharmazie sowie als Sorptionsspeicher insbesondere für Gase, Flüssigkeiten und dergleichen.

Aktivkohle ist aufgrund ihrer recht unspezifischen adsorptiven Eigenschaften das am meisten angewendete Adsorbens. Gesetzliche Auflagen, aber auch das steigende Bewußtsein der Verantwortung für die Umwelt, führen zu einem steigenden Bedarf an Aktivkohle.

Aktivkohle wird im allgemeinen durch Carbonisierung (synonym auch als Schwelung, Pyrolyse, Abbrand etc. bezeichnet) und anschließende Aktivierung kohlenstofflialtiger Ausgangsverbindungen erhalten, wobei solche Ausgangsverbindungen bevorzugt werden, die zu ökonomisch vernünftigen Ausbeuten führen. Denn die Gewichtsverluste durch Abspalten flüchtiger Bestandteile bei der Carbonisierung und durch den nachfolgenden Abbrand bei der Aktivierung sind erheblich. Für weitergehende Einzelheiten der Aktivkohleherstellung kann beispielsweise verwiesen werden auf *H. v. Kienle und E. Bäder*, "Aktivkohle und ihre industrielle Anwendung", Enke Verlag Stuttgart, 1980.

Die Beschaffenheit der erzeugten Aktivkohle - fein- oder grobporig, fest oder brüchig etc. - hängt vom Ausgangsmaterial ab. Übliche Ausgangsmaterialien sind Kokosnußschalen, Holzkohle und Holz (z. B. Holzabfälle), Torf, Steinkohle, Peche, aber auch besondere Kunststoffe, die unter anderem bei der Herstellung von Aktivkohlegeweben eine gewisse Rolle spielen.

Aktivkohle wird in verschiedenen Formen verwendet: Pulverkohle, Splitterkohle bzw. Kornkohle, Formkohle und seit Ende der 1970er Jahre auch kugelförmige Aktivkohle ("Kugelkohle"). Kugelförmige Aktivkohle hat gegenüber anderen Formen von Aktivkohle, wie Pulver-, Splitter-, Korn- und Formkohle und dergleichen, eine Reihe von Vorteilen, die sie für bestimmte Applikationen wertvoll oder sogar unverzichtbar macht: Sie ist rieselfähig, abriebfest bzw. staubfrei und hart. Kugelkohle ist wegen ihrer speziellen Form, aber auch wegen der hohen Abriebfestigkeit beispielsweise für besondere Einsatzgebiete sehr gefragt.

Kugelkohle wird heute noch meist durch mehrstufige und sehr aufwendige Verfahren hergestellt. Das bekannteste Verfahren besteht in der Herstellung von Kügelchen aus Steinkohlenteerpech und geeigneten asphaltartigen Rückständen der Erdölchemie, welche oxidiert werden - damit sie unschmelzbar werden -, und nachfolgend geschwelt und aktiviert werden. Beispielsweise kann die Kugelkohle auch in einem mehrstufigen Verfahren ausgehend von Bitumen hergestellt werden. Diese mehrstufigen Verfahren sind sehr kostenintensiv, und der damit verbundene hohe Preis dieser Kugelkohle verhindert viele Anwendungen, bei denen die Kugelkohle aufgrund ihrer Eigenschaften eigentlich bevorzugt werden müßte.

In der WO 98/07655 A1 wird ein Verfahren zur Herstellung von Aktivkohlekügelchen beschrieben, bei dem zunächst eine Mischung, die einen aus der Diisocyanatherstellung stammenden Destillationsrückstand, einen kohlenstoffhaltigen Verarbeitungshilfsstoff und gegebenenfalls einen oder mehrere weitere Zusatzstoffe umfaßt, zu rieselförmigen Kügelchen verarbeitet wird und anschließend die auf diese Weise erhaltenen Kügelchen carbonisiert und dann aktiviert werden.

Aus dem Stand der Technik bekannt ist ferner die Herstellung von Kugelkohle durch Schwelung und anschließende Aktivierung von neuen oder gebrauchten Ionenaustauschern, die Sulfonsäuregruppen enthalten, bzw. durch Schwelung von Ionenaustauschervorstufen in Gegenwart von Schwefelsäure und anschließende Aktivierung, wobei die Sulfonsäuregruppen bzw. die Schwefelsäure die Funktion eines Vernetzers haben. Solche Verfahren sind beispielsweise in der DE 43 28 219 A1 und in der DE 43 04 026 A1 sowie in der DE 196 00 237 A1 einschließlich der deutschen Zusatzanmeldung DE 196 25 069 A1 beschrieben.

EP 1049116 A1 betrifft ein kohlenstoffhaltiges Material mit einem Gesamtporenvolumen von 0,3 bis 2,0 cm³/g pro Masseeinheit, einem Volumen von Mikroporen mit Durchmessern von 1 bis 2 nm von 10 bis 60 %, bezogen auf das Gesamtporenvolumen, einem Volumen von Mesoporen mit Durchmessern von 1 bis 20 nm von 20 bis 70 %, bezogen auf das Gesamtporenvolumen, einem Volumen von Makroporen mit Durchmessern über 20 nm von nicht mehr als 20 %, bezogen auf das Gesamtporenvolumen, und einer spezifischen Oberfläche von 1.000 bis 2.500 m²/g.

Bei speziellen Anwendungen sind aber nicht nur die Geometrie bzw. die äußere Gestalt der Aktivkohle von entscheidender Bedeutung, sondern auch deren Porosität, insbesondere das Gesamtporenvolumen und die Adsorptionskapazität einerseits und die Verteilung der Poren, d. h. der Anteil an Mikro-, Meso-und Makroporen in bezug auf das Gesamtporenvolumen, andererseits.

Bei einer Reihe von Anwendungen ist eine besonders hohe Meso- und Makroporosität der Aktivkohle, d. h. ein großer Meso- und Makroporenvolumenanteil, bei insgesamt hohem Gesamtporenvolumen gefragt, so beispielsweise bei den eingangs genannten Anwendungen, so z. B. für die Verwendung in der Lebensmittelindustrie, für die Herstellung bestimmter Adsorptionsfiltermaterialien (z. B. für ABC-Schutzbekleidung), für die Adsorption von Giftstoffen, Schadstoffen und Gerüchen, insbesondere aus Gas- bzw. Luftströmen, zur Reinigung oder Aufbereitung von Gasen, wie insbesondere Luft, wie auch Flüssigkeiten, zur Anwendung in der Medizin bzw. Pharmazie, bei der sorptiven Speicherung von Gasen oder Flüssigkeiten und dergleichen.

Die aus dem Stand der Technik zu diesem Zweck bekannte Aktivkohle weist zwar eine gewisse Meso- und Makroporosität auf, die aber nicht in allen Fällen ausreichend ist. Zudem beobachtet man mit zunehmender Porosität eine unerwünschte, bisweilen inakzeptable Abnahme der mechanischen Stabilität bzw. Abriebfestigkeit. Auch sind der Anteil an Meso- und Makroporen am Gesamtporenvolumen und das absolute Porenvolumen nicht immer ausreichend, um für alle Anwendungen eine ausreichende Leistungsfähigkeit und/oder eine ausreichende Imprägnierfähigkeit (z. B. Imprägnierung mit Metallen oder Metallsalzen) zu gewährleisten.

Es ist daher eine Aufgabe der vorliegenden Erfindung, ein insbesondere für die vorgenannten Anwendungsgebiete geeignetes Hochleistungsadsorbens auf der Basis von Aktivkohle bereitzustellen, welches die zuvor geschilderten

Nachteile des Standes der Technik zumindest weitgehend vermeidet oder aber wenigstens abschwächt. Insbesondere sollte das erfindungsgemäß bereitzustellende Adsorbens eine hohe Meso- und Makroporosität, d. h. einen hohen Meso- und Makroporenanteil in bezug auf das Gesamtporenvolumen, sowie ein großes Gesamtporenvolumen aufweisen, dennoch gleichzeitig über eine gute mechanische Beständigkeit, insbesondere eine hohe Abrieb- und Berstbeständigkeit, verfügen.

Im Rahmen der vorliegenden Erfindung bezeichnet der Begriff der Mikroporen solche Poren mit Porendurchmessern von bis zu 20 Å einschließlich, wohingegen der Begriff der Mesoporen solche Poren mit Porendurchmessern im Bereich von mehr als 20 Å (d. h. > 20 Å) bis 500 Å einschließlich bezeichnet und der Begriff der Makroporen solche Poren mit Porendurchmessern von mehr als 500 Å (d. h. > 500 Å) bezeichnet:
- Mikroporen: Porendurchmesser_{Mikroporen} ≥ 20 Å
- Mesoporen: 20 Å < Porendurchmesser_{Mesoporen} ≤ 500 Å
- Makroporen: Porendurchmesser_{Makroporen} > 500 Å

Zur Lösung des zuvor geschilderten Problems schlägt die vorliegende Erfindung - gemäß einem **ersten** Aspekt der vorliegenden Erfindung - Hochleistungsadsorbentien auf der Basis von Aktivkohle in Form von diskreten Aktivkohlekörnern, vorzugsweise in Kugelform, nach Anspruch 1 vor. Weitere, insbesondere vorteilhafte Ausgestaltungen der erfindungsgemäßen Hochleistungsadsorbentien sind Gegenstand der diesbezüglichen Unteransprüche.

Weiterer Gegenstand der vorliegenden Erfindung ist - gemäß einem **zweiten** Aspekt der vorliegenden Erfindung - das erfindungsgemäße Verfahren zur Herstellung der Hochleistungsadsorbentien nach der vorliegenden Erfindung, wie es in den diesbezüglichen Verfahrensansprüchen näher definiert ist.

Wiederum weiterer Gegenstand der vorliegenden Erfindung ist - gemäß einem **dritten** Aspekt der vorliegenden Erfindung - die erfindungsgemäße Verwendung der Hochleistungsadsorbentien nach der vorliegenden Erfindung, wie sie in den diesbezüglichen Verwendungsansprüchen näher definiert ist. Gegenstand der vorliegenden Erfindung sind somit - gemäß einem **ersten** Aspekt der vorliegenden Erfindung - Hochleistungsadsorbentien auf der Basis von Aktivkohle in Form von diskreten Aktivkohlekörnern, vorzugsweise in Kugelform, welche durch die folgenden Parameter gekennzeichnet sind: ein Gesamtporen volumen nach Gurvich von mindestens 0,8 cm³/g,
- einen Anteil an durch Poren mit Porendurchmessern von mehr als 20 Å gebildetem Porenvolumen (d. h. mit anderen Worten einen Anteil an Meso- und Makroporenvolumen) von mindestens 70% des Gesamtporenvolumens der Hochleistungsadsorbentien (Dieser Parameter wird synonym auch als "Anteil des äußeren Porenvolumens in bezug auf das Gesamtporenvolumen" bezeichnet.),
- einen mittleren Porendurchmesser von mehr als 25 Å,
- eine BET-Oberfläche von mindestens 1.250 m²/g,
- eine Iodzahl von mindestens 1.350 mg/g.

Die erfindungsgemäßen Hochleistungsadsorbentien bzw. Aktivkohlen zeichnen sich - neben den vorgenannten Eigenschaften bzw. Parametern, insbesondere einem hohen Meso- und Makroporenvolumenanteil (d. h. einem hohen Anteil an Porenvolumen von Poren mit einem Porendurchmesser von mehr als 20 Å) - weiterhin insbesondere durch eine große Gesamtporosität und eine gleichzeitig große BET-Oberfläche aus.

Wie nachfolgend noch ausgeführt wird, ist trotz der hohen Gesamtporosität auch die mechanische Belastbarkeit, insbesondere die Abriebfestigkeit sowie die Berst- bzw. Druckbelastbarkeit, der erfindungsgemäßen Hochleistungsadsorbentien - im Unterschied zu vergleichbaren hochporösen Aktivkohlen des Standes der Technik - extrem hoch, so daß die erfindungsgemäßen Hochleistungsadsorbentien bzw. Aktivkohlen auch für solche Anwendungen geeignet sind, bei denen sie großen mechanischen Belastungen ausgesetzt sind.

Bei allen vorstehend genannten und noch im folgenden genannten Parameterangaben ist zu beachten, daß die aufgeführten Grenzwerte, insbesondere Ober- und Untergrenzen, mitumfaßt sind, d. h. alle Werteangaben verstehen sich einschließlich der jeweiligen Grenzen, sofern nichts Gegenteiliges im Einzelfall angegeben ist. Weiterhin versteht es sich von selbst, daß es einzelfallbedingt oder anwendungsbezogen gegebenenfalls erforderlich sein kann, geringfügig von den genannten Grenzwerten abzuweichen, ohne daß der Rahmen der vorliegenden Erfindung verlassen ist.

Die vorgenannten und im folgenden noch genannten Parameterangaben werden mit genormten oder explizit angegebenen Bestimmungsverfahren oder aber mit dem Fachmann an sich geläufigen Bestimmungsmethoden bestimmt.

Die Parameterangaben betreffend die Charakterisierung der Porosität, insbesondere des zuvor bezifferten Meso- und Makroporenanteils (d. h. Anteils an Poren mit Porendurchmessern von mehr als 20 Ä in bezug auf das Gesamtporenvolumens der Hochleistungsadsorbentien), ergeben sich jeweils aus der Stickstoffisotherme der vermessenen Aktivkohle.

Die Bestimmung des mittleren Porendurchmessers erfolgt gleichermaßen auf Basis der jeweiligen Stickstoffisothermen.

Die Bestimmung der spezifischen Oberfläche gemäß BET ist dem Fachmann grundsätzlich als solches bekannt, so daß diesbezüglich keine weitergehenden Einzelheiten ausgeführt werden zu brauchen. Alle BET-Oberflächenangaben beziehen sich auf die Bestimmung gemäß ASTM D6556-04. Im Rahmen der vorliegenden Erfindung wird zur Bestimmung der BET-Oberfläche die sogenannte MultiPoint-BET-Bestimmungsmethode (MP-BET) in einem Partialdruckbereich p/p₀ von 0,05 bis 0,1 angewendet.

In bezug auf weitergehende Einzelheiten zur Bestimmung der BET-Oberfläche bzw. zu der BET-Methode kann auf die vorgenannte ASTM D6556-04 sowie auf Römpp Chemielexikon, 10. Auflage, Georg Thieme Verlag, Stuttgart/New York, Stichwort: "BET-Methode", einschließlich der dort referierten Literatur und auf Winnacker-Küchler (3. Auflage), Band 7, Seiten 93 ff. sowie auf Z. Anal. Chem. 238, Seiten 187 bis 193 (1968) verwiesen werden.

Wie zuvor ausgeführt und nachfolgend näher spezifiziert, ist eine Besonderheit der erfindungsgemäßen Hochleistungsadsorbentien unter anderem darin zu sehen, daß sie über ein sehr großes Gesamtporenvolumen nach Gurvich verfügen, so daß eine große Adsorptionskapazität bereitgestellt wird, wobei ein hoher Anteil auf das Meso- und Makroporenvolumen (d. h. also auf das durch Poren mit Porendurchmessern oberhalb von 20 Å gebildete Porenvolumen) entfällt - nämlich mindestens 70% des Gesamtporenvolumens.

Was die Bestimmung des Gesamtporenvolumens nach Gurvich anbelangt, so handelt es sich um eine dem Fachmann auf diesem Gebiet an sich bekannte Meß-Bestimmungsmethode. Zu weitergehenden Einzelheiten bezüglich der Bestimmung des Gesamtporenvolumens nach Gurvich kann beispielsweise verwiesen werden auf L. Gurvich (1915), J. Phys. Chem. Soc. Russ. 47, 805, sowie auf S. Lowell et al., Characterization of Porous Solids and Powders: Surface Area Pore Size and Density, Kluwer Academic Publishers, Article Technology Series, Seiten 111 ff.

Im allgemeinen beträgt das Gesamtporenvolumen nach Gurvich der erfindungsgemäßen Hochleistungsadsorbentien mindestens 0,8 cm³/g, insbesondere mindestens 1,0 cm³/g, vorzugsweise mindestens 1,2 cm³/g, und kann im allgemeinen Werte von bis zu 2,0 cm³/g, insbesondere bis zu 2,5 cm³/g, vorzugsweise bis zu 3,0 cm³/g, besonders bevorzugt bis zu 3,5 cm³/g, erreichen.

Im allgemeinen liegt das Gesamtporenvolumen nach Gurvich der erfindungsgemäßen Hochleistungsadsorbentien im Bereich von 0,8 bis 3,5 cm³/g, insbesondere 1,0 bis 3,5 cm³/g, vorzugsweise 1,2 bis 3,2 cm³/g.

Aufgrund ihrer hohen Meso- und Makroporosität ist das Meso- und Makroporenvolumen der erfindungsgemäßen Hochleistungsadsorbentien (d. h. also mit anderen Worten das durch Poren mit Porendurchmessern von mehr als 20 Å gebildete Porenvolumen) relativ hoch: Im allgemeinen liegt das durch Poren mit Porendurchmessern von mehr als 20 Å gebildete Porenvolumen (d. h. also das Meso- und Makroporenvolumen) nach Carbon Black der erfindungsgemäßen Hochleistungsadsorbentien im Bereich von 0,4 bis 3,3 cm³/g, insbesondere 0,8 bis 3,2 cm³/g, bevorzugt 1,0 bis 3,1 cm³/g, besonders bevorzugt 1,2 bis 3,0 cm³/g, ganz besonders bevorzugt 1,2 bis 2,8 cm³/g. Das durch Poren mit Porendurchmessern von mehr als 20 Å gebildete Porenvolumen wird synonym auch als "äußeres Porenvolumen" bezeichnet.

Im allgemeinen sind mindestens 75 %, ganz besonders bevorzugt mindestens 80 % des Gesamtporenvolumens der erfindungsgemäßen Hochleistungsadsorbentien durch das Porenvolumen von Poren mit Porendurchmessern von mehr als 20 Å (d. h. also mit anderen Worten durch das Meso- und Makroporenvolumen) gebildet.

Im allgemeinen sind 70 % bis 95 %, vorzugsweise 70 % bis 90 %, besonders bevorzugt 70 bis 85 %, des Gesamtporenvolumens der erfindungsgemäßen Hochleistungsadsorbentien durch das Porenvolumen von Poren mit Porendurchmessern von mehr als 20 Å gebildet. Die vorgenannten Prozentangaben bezeichnen somit den Anteil des sogenannten äußeren Porenvolumens (d. h. des durch Poren mit Porendurchmessern von mehr als 20 Å gebildeten Porenvolumens) am Gesamtporenvolumen der erfindungsgemäßen Hochleistungsadsorbentien.

Die Bestimmungsmethode nach Carbon Black ist dem Fachmann an sich bekannt (ebenso wie die diesbezügliche Auswertung, einschließlich Plot und Festlegung des sogenannten p/p₀-Bereiches), so daß es diesbezüglich keiner weitergehenden Einzelheiten bedarf. Zudem kann zu weitergehenden Einzelheiten der Bestimmung der Porenoberfläche und des Porenvolumens nach Carbon Black beispielsweise verwiesen werden auf R. W. Magee, Evaluation of the External Surface Area of Carbon Black by Nitrogen Adsorption, Presented at the Meeting of the Rubber Division of the American Chem. Soc., Oktober 1994, z. B. referiert in: Quantachrome Instruments, AUTOSORB-1, AS1 WinVersion 1.50, Operating Manual, OM, 05061, Quantachrome Instruments 2004, Florida, USA, Seiten 71 ff.

Aufgrund der hohen Meso- und Makroporosität der erfindungsgemäßen Hochleistungsadsorbentien ist der mittlere Porendurchmesser relativ hoch: Im allgemeinen beträgt er mindestens 30 Å, insbesondere mindestens 35 Å, bevorzugt mindestens 40 Å.

Im allgemeinen liegt der mittlere Porendurchmesser der erfindungsgemäßen Hochleistungsadsorbentien im Bereich von 25 bis 75 Å, insbesondere 30 bis 75 Å, vorzugsweise 35 bis 70 Å, besonders bevorzugt 40 bis 65 Å.

Wie zuvor ausgeführt, ist eine weitere Besonderheit der erfindungsgemäßen Hochleistungsadsorbentien in der relativ großen BET-Oberfläche zu sehen, welche mindestens 1.250 m²/g, vorzugsweise mindestens 1.400 m²/g, besonders bevorzugt mindestens 1.500 m²/g, ganz besonders bevorzugt mindestens 1.600 m²/g, beträgt.

Im allgemeinen liegt die BET-Oberfläche der erfindungsgemäßen Hochleistungsadsorbentien im Bereich von 1.250 m²/g bis 2.800 m²/g, insbesondere 1.400 bis 2.500 m²/g, vorzugsweise 1.500 bis 2.300 m²/g, besonders bevorzugt 1.600 bis 2.100 m²/g.

Was die äußere Porenoberfläche nach Carbon Black der erfindungsgemäßen Hochleistungsadsorbentien anbelangt (d. h. also die durch Poren mit Porendurchmessern von mehr als 20 Å gebildete Porenoberfläche), so ist diese auf grund des hohen Meso- und Makroporenanteils relativ groß und liegt im allgemeinen im Bereich von 200 bis 1.000 m²/g, insbesondere 250 bis 950 m²/g, bevorzugt 350 bis 900 m²/g, besonders bevorzugt 400 bis 850 m²/g.

Im allgemeinen bildet die äußere Porenoberfläche nach Carbon Black der erfindungsgemäßen Hochleistungsadsorbentien (d. h. also die durch Poren mit Porendurchmessern von mehr als 20 Å gebildete Porenoberfläche) bis zu 30 %, insbesondere bis zu 40 %, vorzugsweise bis zu 50 %, der Gesamtporenoberfläche der erfindungsgemäßen Hochleistungsadsorbentien. Insbesondere bildet die äußere Porenoberfläche nach Carbon Black der erfindungsgemäßen Hochleistungsadsorbentien (d. h. also die durch Poren mit Porendurchmessern von mehr als 20 Å gebildete Porenoberfläche) 10 bis 50 %, insbesondere 15 bis 45 %, bevorzugt 20 bis 40 %, der Gesamtporenoberfläche der erfindungsgemäßen Hochleistungsadsorbentien.

Darüber hinaus verfügen die erfindungsgemäßen Hochleistungsadsorbentien über eine extrem hohe Butanadsorption und gleichzeitig eine extrem hohe Iodzahl, was ihre Eigenschaft charakterisiert, exzellente Adsorptionseigenschaften in bezug auf verschiedenste zu adsorbierende Stoffe aufzuweisen.

So liegt die gemäß ASTM D5742-95/00 bestimmte Butanadsorption der erfindungsgemäßen Hochleistungsadsorbentien im allgemeinen bei mindestens 30 %, insbesondere bei mindestens 35 %, vorzugsweise bei mindestens 40 %. Im allgemeinen weisen die erfindungsgemäßen Hochleistungsadsorbentien eine nach ASTM D5742-95/00 bestimmte Butanadsorption im Bereich von 30 % bis 80 %, insbesondere 35 bis 75 %, vorzugsweise 40 bis 70 %, auf.

Die nach ASTM D4607-94/99 bestimmte Iodzahl der erfindungsgemäßen Hochleistungsadsorbentien liegt im allgemeinen bei mindestens 1.350 mg/g. Bevorzugterweise weisen die erfindungsgemäßen Hochleistungsadsorbentien eine nach ASTM D4607-94/99 bestimmte Iodzahl im Bereich von 1.350 bis 2.100 mg/g, insbesondere 1.350 bis 2.000 mg/g, vorzugsweise 1.350 bis 1.900 mg/g, auf. Die Iodzahl kann als ein Maß für diejenige verfügbare Oberfläche gewertet werden, welche durch überwiegend größere Mikroporen bereitgestellt wird; die vorgenannten Werte der Iodzahl der erfindungsgemäßen Hochleistungsadsorbentien zeigen, daß die erfindungsgemäßen Hochleistungsadsorbentien gleichzeitig auch über eine hohe Mikroporosität verfügen.

Aufgrund der hohen Meso- und Makroporosität weisen die erfindungsgemäßen Hochleistungsadsorbentien gleichermaßen hohe Methylenblau- und Melasseadsorptionszahlen auf, welche gemeinsam als Maß für diejenige verfügbare Oberfläche gewertet werden können, die überwiegend durch Meso- und Makroporen bereitgestellt wird. So bezieht sich die Methylenblauzahl bzw. der Methylenblauadsorption, welche die Menge an pro definierter Menge der Adsorbentien adsorbiertem Methylenblau unter definierten Bedingungen bezeichnet (d. h. die Anzahl an ml einer Methylenblaustandardlösung, die durch eine definierte Menge trockener und pulverisierter Adsorbentien entfärbt werden), auf größere Mikroporen und überwiegend kleinere Mesoporen und gibt einen Hinweis auf die Adsorptionskapazität der erfindungsgemäßen Hochleistungsadsorbentien in bezug auf Moleküle, welche eine vergleichbare Größe wie Methylenblau besitzen. Dagegen ist die Melassezahl als Maß für die Meso- und Makroporosität zu werten ist und bezeichnet die Menge an Adsorbentien, welche erforderlich ist, um eine Standardmelasselösung zu entfärben, so daß die Melassezahl einen Hinweis auf die Adsorptionskapazität der erfindungsgemäßen Hochleistungsadsorbentien in bezug auf Moleküle gibt, welche eine vergleichbare Größe wie Melasse (im allgemeinen Zuckerrübenmelasse) besitzen. Zusammen können die Methylenblau- und Melassezahl somit als Maß für die Meso- und Makroporosität der erfindungsgemäßen Hochleistungsadsorbentien gewertet werden.

So beträgt der nach der Methode gemäß CEFIC (Conseil Européen des Fédérations des l'Industrie Chimique, Avenue Louise 250, Bte 71, B - 1050 Brüssel, November 1986, European Council of Chemical Manufacturers' Federations, Testmethoden für Aktivkohlen, Ziffer 2.4 "Methylenblauwert", Seiten 27/28) bestimmte Methylenblauwert der erfindungsgemäßen Hochleistungsadsorbentien mindestens 15 ml, insbesondere mindestens 17 ml, vorzugsweise mindestens 19 ml, und liegt im allgemeinen im Bereich von 15 bis 60 ml, insbesondere 17 bis 50 ml, vorzugsweise 19 bis 45 ml.

Der Methylenblauwert nach der vorgenannten CEFIC-Methode ist somit definiert als die Anzahl von ml einer Methylenblaustandardlösung, welche durch 0,1 g trockener und pulverisierter Aktivkohle entfärbt werden. Für die Durchführung dieser Methode wird ein Glasgefäß mit Schliffstopfen, ein Filter sowie eine Methylenblaustandardlösung benötigt, welche wie folgt hergestellt wird: Eine Menge von 1.200 mg reinen Farbstoffes Methylenblau (entsprechend ca. 1,5 g Methylenblau nach DAB VI [Deutsches Arzneibuch, 6. Ausgabe] oder gleichwertiges Produkt) werden in Wasser in einem 1.000-ml-Meßkolben gelöst, und man läßt die Lösung mehrere Stunden oder über Nacht stehen; zur Überprüfung werden 5,0 ml der Lösung mit 0,25 % (Volumenanteile) Essigsäure in einem Meßkolben auf 1,0 1 aufgefüllt, und danach wird die Extinktion bei 620 nm und 1 cm Schichtdicke gemessen, wobei sie (0,840 ± 0,010) betragen muß. Ist die Extinktion höher, muß mit der berechneten Wassermenge verdünnt werden; falls sie niedriger liegt, wird die Lösung verworfen und neu angesetzt. Zur Probenvorbereitung werden die Hochleistungsadsorbentien in Form körniger Aktivkohle pulverisiert (< 0,1 mm) und dann bei 150 °C zur Gewichtskonstanz getrocknet. Exakt 0,1 g der Kugelkohle werden mit 25 ml (5 ml) der Methylenblaustandardlösung in einem Glasschliffkolben zusammengebracht (Es muß in einem Vortest ermittelt werden, ob eine Anfangszugabe von 25 ml Methylenblaustandardlösung mit 5 ml-Zugaben oder eine Anfangszugabe von 5 ml Methylenblaustandardlösung mit 1-ml-Zugaben verwendet werden kann.). Es wird geschüttelt, bis Entfärbung eintritt. Dann werden weitere 5 ml (1 ml) der Methylenblaustandardlösung zugegeben, und es wird bis zur Entfärbung geschüttelt. Die Zugabe von Methylenblaustandardlösung wird in 5-ml-Mengen (1-ml-Mengen) so lange wiederholt, wie innerhalb von 5 Minuten noch Entfärbung eintritt. Das gesamte Volumen der Testlösung, die durch die Probe entfärbt wurde, wird notiert. Man wiederholt den Test, um die erhaltenen Ergebnisse zu bestätigen. Das Volumen der Methylenblaustandardlösung in ml, die gerade noch entfärbt werden, ist der Methylenblauwert der Hochleistungsadsorbentien. Es ist in diesem Zusammenhang darauf hinzuweisen, daß der Farbstoff Methylenblau nicht getrocknet werden darf, da er wärmeempfindlich ist; der Wassergehalt muß vielmehr rein rechnerisch korrigiert werden.

Die dimensionslose Melassezahl dagegen kann grundsätzlich entweder nach der Norit-Methode (Norit N.V., Amersfoort, Niederlande, Norit-Standardmethode NSTM 2.19 "*Molasses Number (Europe)*") oder alternativ nach der PACS-Methode (PACS = Professional Analytical and Consulting Services Inc., Coraopolis Pennsylvania, USA) bestimmt werden. Im Rahmen der vorliegenden Erfindung werden die Werte für die Melassezahl nach der PACS-Methode ermittelt. So beträgt die nach der PACS-Methode bestimmte Melassezahl der erfindungsgemäßen Hochleistungsadsorbentien mindestens 300, insbesondere mindestens 350, vorzugsweise mindestens 400, und liegt im allgemeinen im Bereich von 300 bis 1.400, insbesondere 350 bis 1.300, vorzugsweise 400 bis 1.250, ganz besonders bevorzugt 700 bis 1.200.

Bei der Bestimmung der Melassezahl nach der Norit- oder PACS-Methode wird diejenige Menge an pulverisiertem Hochleistungsadsorbentien auf Basis von Aktivkohle bestimmt, welche erforderlich ist, um eine Standardmelasselösung zu entfärben. Die Bestimmung erfolgt photometrisch, wobei die Einstellung der Melassestandardlösung gegen eine standardisierte Aktivkohle mit einer Melassezahl von 245 und/oder 350 erfolgt. Für weitergehende diesbezügliche Einzelheiten kann auf die beiden vorgenannten Vorschriften verwiesen werden.

Trotz der hohen Porosität, insbesondere Meso- und Makroporosität, weisen die erfindungsgemäßen Hochleistungsadsorbentien eine hohe Druck- bzw. Berstfestigkeit (Gewichtsbelastbarkeit) sowie eine extrem hohe Abriebfestigkeit auf.

So liegt die Druck- bzw. Berstfestigkeit (Gewichtsbelastbarkeit) pro Aktivkohlekom, insbesondere pro Aktivkohlekügelchen, bei mindestens 5 Newton, insbesondere mindestens 10 Newton, vorzugsweise mindestens 15 Newton. Im allgemeinen variiert die Druck- bzw. Berstfestigkeit (Gewichtsbelastbarkeit) pro Aktivkohlekorn, insbesondere pro Aktivkohlekügelchen, im Bereich von 5 bis 50 Newton, insbesondere 10 bis 45 Newton, vorzugsweise 15 bis 40 Newton.

Wie zuvor ausgeführt, ist auch die Abriebshärte der erfindungsgemäßen Hochleistungsadsorbentien extrem hoch: So liegt die Abriebfestigkeit nach der Methode gemäß CEFIC (Conseil Européen des Fédérations des l'Industrie Chimique, Avenue Louise 250, Bte 71, B - 1050 Brüssel, November 1986, European Council of Chemical Manufacturers' Federations, Testmethoden für Aktivkohlen, Ziffer 1.6 "Mechanische Härte", Seiten 18/19) stets bei 100 % oder nahezu 100 %. Auch nach ASTM D3802 werden Abriebfestigkeiten der erfindungsgemäßen Hochleistungsadsorbentien von stets 100 % oder nahezu 100 % erhalten.

Daher hat die Anmelderin eine modifizierte Testmethode in Anlehnung an diese CEFIC-Methode entwickelt, um aussagekräftigere Werte zu erhalten. Die modifizierte Bestimmungsmethode simuliert besser den Widerstand der Probe bzw. der Hochleistungsadsorbentien gegenüber Abrieb oder Zerreiben unter praxisnahen Bedingungen. Zu diesem Zweck wird die Probe eine definierte Zeit lang in einem horizontal schwingenden, mit einer Wolframcarbidkugel beschickten Mahlbecher unter genormten Bedingungen beansprucht. Zu diesem Zweck wird wie folgt vorgegangen: 200 g einer Probe werden eine Stunde lang bei (120 ± 2) °C im Umlufttrockenschrank (Typ: Heraeus UT 6060 der Fa. Kendro GmbH, Hanau) getrocknet und anschließend in einem Exsikkator über Trockenmittel auf Raumtemperatur abgekühlt. 50 g der getrockneten Probe werden entnommen und mittels einer Siebmaschine mit Analysensieb (z. B. Typ: AS 200 control der Fa. Retsch GmbH, Hanau) bei einer Schwinghöhe von 1,2 mm zehn Minuten lang über ein Analysensieb abgesiebt, wobei das Analysensieb in Abhängigkeit von der Kornverteilung der zu vermessenden Probe ausgewählt wird (z. B. Analysensieb der Maschenweite: 0,315 mm, Durchmesser: 200 mm, Höhe: 50 mm); das Unterkorn wird verworfen. 5 ml des Nennkornes werden in einen 10-ml-Meßzylinder nach DIN ISO 384 (Volumen: 10 ml, Höhe: 90 mm) abgefüllt und das Gewicht mittels eines Wägeglases mit eingeschliffenem Glasdeckel (Volumen: 15 ml, Durchmesser: 35 mm, Höhe: 30 mm) auf 0,1 mg genau bestimmt mittels Analysenwaage (Typ: BP121S der Sartorius AG, Göttingen, Wägebereich: 120 g, Genauigkeitsklasse: E2, Ablesbarkeit: 0,1 mg). Die abgewogene Probe wird zusammen mit einer Wolframcarbidmahlkugel mit 20 mm Durchmesser in einen 25-ml-Mahlbecher mit Schraubverschluß (Volumen: 25 ml, Durchmesser: 30 mm, Länge: 65 mm, Werkstoff: Edelstahl) gegeben und dann der Abriebtest mittels Schwingmühle (Typ: MM301 der Fa. Retsch GmbH, Haan, Schwingmühle mit Mahlbecher) durchgeführt; dabei schwingt der Mahlbecher in horizontaler Lage eine Minute lang mit einer Frequenz von 10 Hz in der Schwingmühle, was dazu führt, daß die Mahlkugel auf die Probe einschlägt und somit Abrieb erzeugt. Anschließend wird die Probe mittels einer Siebmaschine bei einer Schwinghöhe von 1,2 mm fünf Minuten lang über das vorgenannte Analysensieb abgesiebt, wobei das Unterkorn wieder verworfen und das Nennkorn, welches abhängig von der Kornverteilung der betreffenden Probe ist (z. B. Nennkorn größer 0,315 mm) auf 0,1 mg genau im Wägeglas mit Deckel zurückgewogen wird. Die Berechnung der Abriebhärte erfolgt als Masseanteil in % nach folgender Formel: Abriebhärte [%] = (100 x Rückwaage [g]) / Einwaage [g].

Gemäß dieser von der Anmelderin modifizierten Bestimmungsmethode in Abwandlung der vorgenannten CEFIC-Norm beträgt die Abriebfestigkeit der erfindungsgemäßen Hochleistungsadsorbentien mindestens 75 %, insbesondere mindestens 80 %, vorzugsweise mindestens 85 %, besonders bevorzugt mindestens 90 %, ganz besonders bevorzugt mindestens 95 %.

Wie zuvor ausgeführt, ist eine weitere Besonderheit der erfindungsgemäßen Hochleistungsadsorbentien darin zu sehen, daß sie auch über eine gewisse Mikroporosität und somit auch über eine gewisse Mikroporenoberfläche (d. h. Oberfläche, welche durch Poren mit Porendurchmesser von ≤ 20 Å gebildet ist) verzügen. Im allgemeinen beträgt die durch Poren mit Porendurchmessern von ≤ 20 Å gebildete Mikroporenoberfläche nach Carbon Black der erfindungsgemäßen Hochleistungsadsorbentien mindestens 1.000 m²/g, insbesondere mindestens 1.100 m²/g, vorzugsweise mindestens 1.200 m²/g, und liegt im allgemeinen im Bereich von 1.000 bis 1.800 m²/g, insbesondere 1.100 bis 1.600 m²/g, vorzugsweise 1.200 bis 1.500 m²/g.

Im allgemeinen beträgt die durch Poren mit Porendurchmessern von ≤ 20 Å gebildete Mikroporenoberfläche nach Carbon Black der erfindungsgemäßen Hochleistungsadsorbentien mindestens 30 %, insbesondere mindestens 40 %, vorzugsweise mindestens 50 %, der Gesamtporenoberfläche der erfindungsgemäßen Hochleistungsadsorbentien. Insbesondere liegt die durch Poren mit Porendurchmessern von ≤ 20 Å gebildete Mikroporenoberfläche nach Carbon Black der erfindungsgemäßen Hochleistungsadsorbentien im Bereich von 50 bis 90 %, insbesondere 55 bis 85 %, bevorzugt 60 bis 80 %, der Gesamtporenoberfläche der erfindungsgemäßen Hochleistungsadsorbentien.

Auch das gewichts- und volumenbezogene Volumen V_{ads} (N₂) der erfindungsgemäßen Hochleistungsadsorbentien bei unterschiedlichen Partialdrücken p/p₀ ist sehr groß:

So beträgt das gewichtsbezogene adsorbierte N₂-Volumen V_{ads (gew)} der erfindungsgemäßen Hochleistungsadsorbentien, bestimmt bei einem Partialdruck p/p₀ von 0,25, mindestens 300 cm³/g, insbesondere mindestens 350 cm³/g, vorzugsweise mindestens 375 cm³/g, und liegt insbesondere im Bereich von 300 bis 800 cm³/g, vorzugsweise 350 bis 700 cm³/g, besonders bevorzugt 375 bis 650 cm³/g_{.}

Im allgemeinen beträgt das volumenbezogene adsorbierte N₂-Volumen V_{ads (vol.)} der erfindungsgemäßen Hochleistungsadsorbentien, bestimmt bei einem Partialdruck p/p₀ von 0,25, mindestens 75 cm³/cm³, insbesondere mindestens 100 cm³/cm³, und liegt insbesondere im Bereich von 75 bis 300 cm³/cm³, vorzugsweise 80 bis 275 cm³/cm³, besonders bevorzugt 90 bis 250 cm/cm³.

Im allgemeinen beträgt das gewichtsbezogene adsorbierte N₂-Volumen V_{ads (gew.)} der erfindungsgemäßen Hochleistungsadsorbentien, bestimmt bei einem Partialdruck p/p₀ von 0,995, mindestens 400 cm³/g, insbesondere mindestens 450 cm³/g, und liegt insbesondere im Bereich von 400 bis 2.300 cm³/g, vorzugsweise 450 bis 2.200 cm³/g, besonders bevorzugt 750 bis 2.100 cm³/g.

Im allgemeinen beträgt das volumenbezogene adsorbierte N₂- Volumen V_{ads (vol.)} der erfindungsgemäßen Hochleistungsadsorbentien, bestimmt bei einem Partialdruck p/p₀ von 0,995, mindestens 200 cm³/cm³, insbesondere mindestens 250 cm³/cm³, und liegt insbesondere im Bereich von 200 bis 500 cm³/cm³, vorzugsweise 250 bis 400 cm³/cm³, besonders bevorzugt 275 bis 380 cm³/cm³.

Die erfindungsgemäßen Hochleistungsadsorbentien sind auf Basis komförmiger, insbesondere kugelförmiger, Aktivkohle ausgebildet, deren mittlerer Teilchendurchmesser, bestimmt nach ASTM D2862-97/04, im allgemeinen im Bereich von 0,01 bis 2,0 mm, insbesondere 0,01 bis 1,0 mm, vorzugsweise 0,05 bis 0,9 mm, besonders bevorzugt 0,1 bis 0,8 mm, ganz besonders bevorzugt 0,15 bis 0,7 mm, liegt.

Der Aschegehalt der erfindungsgemäßen Hochleistungsadsorbentien, bestimmt nach ASTM D2866-94/04, beträgt höchstens 1 %, insbesondere höchstens 0,8 %, vorzugsweise höchstens 0,6 %, besonders bevorzugt höchstens 0,5 %.

Der nach ASTM D2867-04/04 bestimmte Feuchtigkeitsgehalt der erfindungsgemäßen Hochleistungsadsorbentien beträgt höchstens 1 %, insbesondere höchstens 0,5 %, vorzugsweise höchstens 0,2 %.

Die erfindungsgemäßen Hochleistungsadsorbentien weisen im allgemeinen eine Schüttdichte (Bulk Density), bestimmt nach ASTM B527-93/00, im Bereich von 150 bis 750 g/l, insbesondere 175 bis 650 g/l, vorzugsweise 200 bis 600 g/l, auf.

Gemäß einer besonderen Auswhrungsform der vorliegenden Erfindung sind Gegenstand der vorliegenden Erfindung Hochleistungsadsorbentien auf der Basis von Aktivkohle in Form von diskreten Aktivkohlekörnern, vorzugsweise in Kugelform, insbesondere wie zuvor beschieben, welche durch die folgenden Parameter gekennzeichnet sind:
- einen Anteil an durch Poren mit Porendurchmessern von mehr als 20 Å gebildetem Porenvolumen von mindestens 70 % des Gesamtporenvolumens der Hochleistungsadsorbentien,
- einen mittleren Porendurchmesser von mehr als 25 Å,
- eine BET-Oberfläche von mindestens 1.250 m²/g,
- einen Methylenblauwert von mindestens 15 ml und
- eine Melassezahl von mindestens 300.

Weiterer Gegenstand der vorliegenden Erfindung - gemäß einem **zweiten** Aspekt der vorliegenden Erfindung - ist das erfindungsgemäße Verfahren zur Herstellung der Hochleistungsadsorbentien nach der vorliegenden Erfindung. Gegenstand der vorliegenden Erfindung gemäß diesem Erfindungsaspekt ist somit ein Verfahren zur Herstellung der zuvor beschriebenen Hochleistungsadsorbentien auf der Basis von Aktivkohle, bei dem ein kohlenstoffhaltiges Ausgangsmaterial in Form von sulfonierten Styrol/Divinylbenzol-Copolymeren zunächst carbonisiert und nachfolgend aktiviert wird, wobei die Aktivierung zweistufig durchgefiihrt wird, wobei das carbonisierte Ausgangsmaterial zunächst in einem ersten Aktivierungsschritt einer Aktivierung in einer wasserdampfhaltigen Atmosphäre unterzogen wird, gefolgt von einem zweiten Aktivierungsschritt der Aktivierung in einer CO₂-haltigen Atmosphäre.

Was die für die Herstellung der erfindungsgemäßen Hochleistungsadsorbentien eingesetzten kohlenstoffhaltigen Ausgangsmaterialien anbelangt, so können insbesondere sulfonierte divinylbenzolvernetzten Polystyrole, vorzugsweise in Kornform, besonders bevorzugt in Kugelform, zum Einsatz kommen. Der Divinylbenzolgehalt der als Ausgangsmaterialien zur Herstellung der erfindungsgemäßen Hochleistungsadsorbentien verwendeten sulfonierten Styrol/Divinylbenzol-Copolymere sollte insbesondere im Bereich von 1 bis 20 Gew.-%, insbesondere 1 bis 15 Gew.-%, vorzugsweise 2 bis 10 Gew.-%, bezogen auf die Styrol/Divinylbenzol-Copolymere, liegen. Die Ausgangscopolymere können grundsätzlich vom Geltyp oder aber vom makroporösen Typ ausgewählt sein. Wenn von unsulfonierten Ausgangsmaterialien ausgegangen wird, kann die Sulfonierung in situ (insbesondere vor und/oder während der Carbonisierung) durchgeführt werden, insbesondere mit dem Fachmann an sich bekannten Methoden, vorzugsweise mittels Schwefelsäure und/oder Oleum und/oder SO₃; dies ist dem Fachmann an sich geläufig (vgl. hierzu auch der eingangs geschilderte Stand der Technik). Als Ausgangsmaterialien besonders bewährt haben sich gelförmige oder makroporöse Typen der entsprechenden Ionenaustauscherharze bzw. der entsprechenden unsulfonierten Vorstufen von Ionenaustauscherharzen, welche noch sulfoniert werden müssen.

Bei der Carbonisierung (synonym auch als Pyrolyse, Abbrand oder Schwelung bezeichnet) erfolgt die Umwandlung der kohlenstoffhaltigen Ausgangspolymere zu Kohlenstoff, d. h. mit anderen Worten wird das kohlenstoffhaltige Ausgangsmaterial verkohlt. Bei der Carbonisierung der zuvor genannten organischen Polymerkörner, insbesondere Polymerkügelchen, auf Basis von Styrol und Divinylbenzol, die Sulfonsäuregruppen enthalten, führt die Abspaltung der Sulfonsäuregruppen während der Carbonisierung zu freien Radikalen und somit zu Vernetzungen, ohne die es keinen Pyrolyserückstand (= Kohlenstoff) gäbe. Im allgemeinen wird die Carbonisierung unter inerter Atmosphäre (z. B. Stickstoff) oder allenfalls leicht oxidierender Atmosphäre durchgefiihrt. Gleichermaßen kann es vorteilhaft sein, während der Carbonisierung, insbesondere bei höheren Temperaturen (z. B. im Bereich von etwa 500 bis 650 °C), zu der Inertatmosphäre eine kleinere Menge an Sauerstoff, insbesondere in Form von Luft (z. B. 1 bis 5 %), zuzugeben, um eine Oxidation des carbonisierten Polymerskeletts zu bewirken und auf diese Weise die nachfolgende Aktivierung zu erleichtern. Im allgemeinen wird die Carbonisierung bei Temperaturen von 100 bis 950 °C, insbesondere 150 bis 900 °C, vorzugsweise 300 bis 850 °C, durchgeführt. Die Gesamtdauer der Carbonisierung liegt dabei etwa bei 30 Minuten bis etwa 10 Stunden, insbesondere etwa 1 Stunde bis etwa 6 Stunden.

Im Anschluß an die Carbonisierung wird das carbonisierte Zwischenprodukt einer Aktivierung unterzogen, am Ende derer die erfindungsgemäßen Hochleistungsadsorbentien auf Basis von Aktivkohle in Kornform, insbesondere Kugelform, resultieren. Das Grundprinzip der Aktivierung besteht darin, einen Teil des bei der Carbonisierung generierten Kohlenstoffs selektiv und gezielt unter geeigneten Bedingungen abzubauen. Hierdurch entstehen zahlreiche Poren, Spalten und Risse, und die auf die Masseneinheit bezogene Oberfläche nimmt erheblich zu. Bei der Aktivierung wird also ein gezielter Abbrand der Kohle vorgenommen. Da bei der Aktivierung Kohlenstoff abgebaut wird, tritt bei diesem Vorgang ein Substanzverlust ein, welcher - unter optimalen Bedingungen - gleichbedeutend mit einer Erhöhung der Porosität und Zunahme der inneren Oberfläche und des Porenvolumens ist. Die Aktivierung erfolgt daher unter selektiven bzw. kontrollierten oxidierenden Bedingungen.

Die Besonderheit bei der Herstellung der erfindungsgemäßen Hochleistungsadsorbentien ist - neben der Auswahl des zuvor beschriebenen Ausgangsmaterials - in der speziellen Verfahrensführung der Aktivierung zu sehen, insbesondere in der Zweistufigkeit der Aktivierung, wobei das carbonisierte Ausgangsmaterial zunächst in einem ersten Aktivierungsschritt einer Aktivierung in einer wasserdampflialtigen Atmosphäre unterzogen wird und sich hieran ein zweiter Aktivierungsschritt in einer CO₂-haltigen Atmosphäre anschließt. Wie die Untersuchungen der Anmelderin ergeben haben, führt überraschenderweise nur die getrennte Durchführung dieser Aktivierungsschritte in der vorgenannten Reihenfolge zu den gewünschten Produkten. Die Umkehr der Reihenfolge der Aktivierungsschritte oder ein gemeinsam durchgeführter Aktivierungsschritt in einer Wasserdampf/Kohlendioxid-Atmosphäre führt dagegen zu deutlich weniger leistungsstarken Produkten, welche nicht die gewünschten Eigenschaften, insbesondere nicht die hohe Gesamtporosität bei hohem Meso/Makroporenanteil und relativ hohem absolutem Mikroporenvolumen sowie hoher mechanischer Stabilität, aufweisen. Wie die Untersuchungen der Anmelderin überraschend gezeigt haben, führt bei erfindungsgemäßer Verfahrensführung die Wasserdampfaktivierung vorwiegend zur Ausbildung des Mikroporenanteils, während die Kohledioxidaktivierung vorwiegend zur Ausbildung der Meso- und Makroporen beiträgt, wobei überraschenderweise die Ausbildung des Meso- und Makroporenvolumens nicht zu Lasten des Mikroporenvolumens oder vice versa erfolgt. Neu und überraschend ist somit insgesamt, daß auf das Weise ein sehr großes Gesamtporenvolumen bei sehr hoher Stabilität und Abriebfestigkeit sowie sehr hohem Meso- und Makroporenanteil bei gleichzeitig hohem Mikroporenanteil erreicht werden kann (d. h. die Ausbildung von Meso- und Makroporen in der enormen Ausprägung führt bei den erfindungsgemäßen Produkten nicht zu einer Reduzierung des Mikroporenanteils, wie im Stand der Technik üblich). Vielmehr wird ein hoher Mikroporenanteil bei gleichzeitig hohem Meso-/Makroporenvolumenanteil erreicht. Im allgemeinen wird derart verfahren, daß der erste Aktivierungsschritt bei Temperaturen von 700 bis 1.300 °C, insbesondere 800 bis 1.200 °C, vorzugsweise 850 bis 950 °C, und/oder über eine Dauer von 5 bis 24 Stunden, vorzugsweise 5 bis 15 Stunden, insbesondere 6 bis 12 Stunden, durchgerührt wird. Üblicherweise kann die Dauer der ersten Aktivierungsstufe in Abhängigkeit von der Erreichung einer vorgegebenen Iodzahl gesteuert werden; beispielsweise kann die erste Aktivierungsstufe bis zur Erreichung einer Iodzahl von mindestens 1.000 mg/g, insbesondere mindestens 1.250 mg/g, durchgeführt werden. Die Atmosphäre der ersten Aktivierungsstufe umfaßt Wasserdampf, insbesondere ein Gemisch aus Wasserdampf/Inertgas, vorzugsweise ein Gemisch aus Wasserdampf/Stickstoff, oder besteht hieraus. Aus den vorgenannten Gründen ist im Rahmen der ersten Aktivierungsstufe die Anwesenheit von anderen Aktivierungsgasen als Wasserdampf, insbesondere die Anwesenheit von Kohlenstoffoxiden (z. B. CO₂), Sauerstoff und/oder Ammoniak, auszuschließen. Gute Ergebnisse werden erhalten, wenn der Durchsatz bzw. die eingesetzte Menge an Wasserdampf 25 bis 350 1/h, insbesondere 50 bis 300 1/h, beträgt, berechnet als Wasser (d. h. flüssiges Wasser bei 25 °C und unter Atmosphärendruck). In Abhängigkeit von der Menge an zu aktivierendem Ausgangsmaterial (= zuvor im Rahmen der Carbonisierung hergestelltes Carbonisat) sollte die eingesetzte Menge bzw. der massenbezogene Durchsatz an Wasserdampf vorteilhafterweise 0,01 bis 50 1/(h - kg), insbesondere 0,02 bis 25 l/(h · kg), vorzugsweise 0,02 bis 5 1/(h - kg), betragen, berechnet als Wasser (d. h. flüssiges Wasser bei 25 °C und unter Atmosphärendruck) und bezogen auf mit Wasserdampf zu aktivierendes Ausgangsmaterial.

Was den zweiten Aktivierungsschritt anbelangt, so wird im allgemeinen derart verfahren, daß der zweite Aktivierungsschritt bei Temperaturen von 700 bis 1.300 °C, insbesondere 800 bis 1.200 °C, vorzugsweise 850 bis 950 °C, und/oder über eine Dauer von 1 bis 10 Stunden, insbesondere 3 bis 8 Stunden, durchgeführt wird. Die Atmosphäre der zweiten Aktivierungsstufe umfaßt CO₂ insbesondere reines CO₂ oder ein Gemisch aus CO₂/Inertgas, insbesondere ein Gemisch aus CO₂/Stickstoff, oder besteht hieraus, wobei reines Kohlendioxid besonders bevorzugt ist. Aus den vorgenannten Gründen ist im Rahmen der zweiten Aktivierungsstufe die Anwesenheit von anderen Aktivierungsgasen als CO₂, insbesondere die Anwesenheit von Wasserdampf, auszuschließen. Gute Ergebnisse werden erhalten, wenn der Durchsatz bzw. die eingesetzte Menge an CO₂ 10 bis 250 m³/h, insbesondere 20 bis 200 m³/h, beträgt (bezogen auf reines CO₂). In Abhängigkeit von der Menge an zu aktivierendem Ausgangsmaterial sollte die eingesetzte Menge bzw. der massenbezogene Durchsatz an CO₂ vorteilhafterweise 0,001 bis 100 m³/(h· kg), insbesondere 0,01 bis 50 m³/(h· kg), vorzugsweise 0,05 bis 10 m³/(h kg), betragen, berechnet als reines gasförmiges CO₂ unter Aktivierungsbedingungen, insbesondere bei dem jeweiligen Druck und bei der jeweiligen Temperatur, welche für die Aktivierung ausgewählt werden, und bezogen auf mit CO₂ zu aktivierendes Ausgangsmaterial.

Üblicherweise wird derart verfahren, daß die erste und zweite Aktivierungsstufe ineinander übergehen (z. B. durch Änderung der Aktivierungsatmosphäre innerhalb derselben Apparatur).

Insbesondere ist es überraschend, daß zum einen die erfindungsgemäße Durchführung der Aktivierung eine exakte Steuerung der Porosität im Hinblick auf den Mikro-, Meso- und Makroporenanteil ermöglicht und zum anderen trotz der hohen Porosität bei gleichzeitig hoher Meso- und Makroporosität und auch guter Mikroporosität eine extrem hohe Abriebfestigkeit und mechanische Druckfestigkeit resultiert. Es war nicht zu erwarten, daß auf diese Weise selektiv eine hohe Meso- und Makroporosität bei gleichzeitig ausreichender Mikroporosität generiert wird.

Durch Variation der zuvor spezifizierten Aktivierungsbedingungen kann gezielt die Porosität eingestellt bzw. gesteuert werden. Auf diese Weise können die erfindungsgemäßen Hochleistungsadsorbentien sozusagen maßgeschneidert werden. Hochleistungsadsorbentien auf der Basis von Aktivkohle mit einer hohen Meso- und Makroporosität bei gleichzeitig guter Mikroporosität und gleichzeitig hoher Stabilität und Abriebfestigkeit sind aus dem Stand der Technik nicht bekannt. Ebenso hervorzuheben ist das exzellente Adsorptionsverhalten gegenüber Molekülen nahezu beliebiger Molekülgröße infolge des Vorhandseins aller Arten von Poren in relativ großen Mengen bzw. Anteilen. Gleichermaßen hervorzuheben ist auch die ausgezeichnete Imprägnierbarkeit der erfindungsgemäßen Produkte mit Katalysatoren bzw. Metallen oder Metallsalzen.

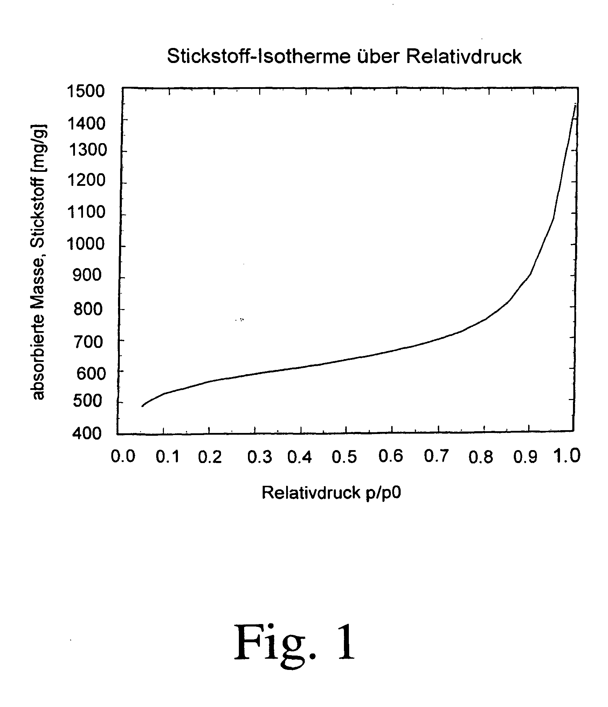

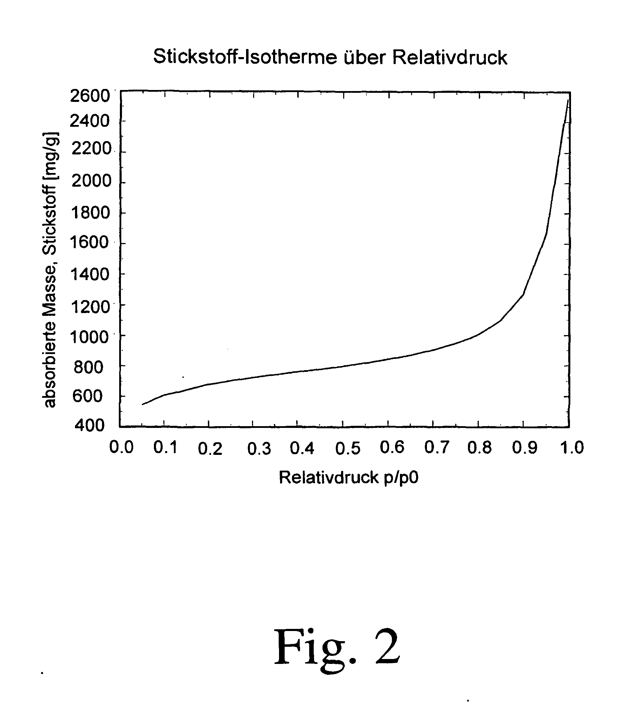

Die Figurendarstellungen gemäß Fig. 1 und Fig. 2 zeigen N₂-Adsorptionsisothermen für zwei unterschiedliche erfindungsgemäße Hochleistungsadsorbentien, welche unter unterschiedlichen Aktivierungsbedingungen hergestellt worden sind. Die physikochemischen Eigenschaften der beiden erfindungsgemäßen Hochleistungsadsorbentien sind zudem in der nachfolgenden Tabelle 1 zusammengefaßt. Zum Vergleich ist dort auch eine handelsübliche Aktivkohle der Fa. Kureha mit den betreffenden physikochemischen Eigenschaften aufgeführt.

Die in Tabelle 1 wiedergegebenen Daten zeigen die Überlegenheit der erfindungsgemäßen Hochleistungsadsorbentien gegenüber einer Aktivkohle des Standes der Technik: Die Kombination einer hohen Gesamtporosität mit hohem Meso-/Makroporenvolumenanteil bei hoher BET-Oberfläche und gleichzeitig guter absoluter Mikroporosität, hoher mechanischer Beständigkeit und exzellenten Adsorptionseigenschaften ist in dieser Kombination - neben den übrigen physikochemischen Parametern - nur bei der erfindungsgemäßen Hochleistungsadsorbentien zu finden. Erfindungsgemäß lassen sich also Hochleistungsadsorbentien auf Basis von Aktivkohle in Kornform, insbesondere Kugelform, herstellen, welche den handelsüblichen Produkten überlegen sind.

Die in Tabelle 1 aufgeführten erfindungsgemäßen Hochleistungsadsorbentien "Aktivkohle I" und "Aktivkohle II" werden jeweils wie folgt hergestellt: Handelsübliche, getrocknete Ionenaustauschervorstufen auf der Basis von divinylbenzolvemetzten Polystyrolcopolymeren mit einem Divinylbenzolgehalt von etwa 4 % werden bei Temperaturen von 100 °C bis 150 °C mit einer Schwefelsäure/Oleum-Mischung in an sich bekannter Weise sulfoniert. Dann wird in an sich bekannter Weise bei Temperaturen bis zu 850 °C vier Stunden lang unter Stickstoffatmosphäre carbonisiert und anschließend die Aktivierung eingeleitet. Während bei der Herstellung der erfindungsgemäßen Aktivkohle I die erste Aktivierungsstufe ("Wasserdampfaktivierung") für eine Dauer von etwa 8,5 Stunden bei etwa 900 °C mit einem Wasserdampfdurchsatz von etwa 100 m³/h und die zweite Aktivierungsstufe ("Kohlendioxidaktivierung") für eine Dauer von etwa 8,0 Stunden bei etwa 900 °C mit einem Kohlendioxiddurchsatz von etwa 35 m³/h durchgeführt wurden, wurden dagegen bei der Herstellung der erfindungsgemäßen Aktivkohle II die erste Aktivierungsstufe ("Wasserdampfaktivierung") für eine Dauer von etwa 10,5 Stunden bei etwa 925 °C mit einem Wasserdampfdurchsatz von etwa 125 m³/h und die zweite Aktivierungsstufe ("Kohlendioxidaktivierung") für eine Dauer von etwa 8 Stunden bei etwa 925 °C mit einem Kohlendioxiddurchsatz von etwa 40 m³/h durchgeführt. Nach Abkühlung auf Raumtemperatur werden die in Tabelle 1 aufgeführten erfindungsgemäßen Produkte erhalten.

Wiederum weiterer Gegenstand der vorliegenden Erfindung - gemäß einem **dritten** Aspekt der vorliegenden Erfindung - ist die erfindungsgemäße Verwendung der Hochleistungsadsorbentien nach der vorliegenden Erfindung.

So eignen sich die erfindungsgemäßen Hochleistungsadsorbentien insbesondere für die Adsorption von Giftstoffen, Schadstoffen und Gerüchen, beispielsweise aus Gas- bzw. Luftströmen. Weiterhin eignen sich die erfindungsgemäßen Hochleistungsadsorbentien auch zur Reinigung und Aufbereitung von Gasen, insbesondere zur Reinigung von Luft, sowie von Flüssigkeiten, wie insbesondere Wasser (z. B. Trinkwasseraufbereitung). Insbesondere eignen sich die erfindungsgemäßen Hochleistungsadsorbentien für die Imprägnierung (z. B. mit Katalysatoren bzw. Metallen oder Metallsalzen).

Des weiteren eignen sich die erfindungsgemäßen Hochleistungsadsorbentien beispielsweise auch zur Verwendung für die bzw. in der Lebensmittelindustrie, insbesondere zur Aufbereitung und/oder Entfärbung von Lebensmitteln.

Weiterhin können die erfindungsgemäßen Hochleistungsadsorbentien auch in Adsorptionsfiltermaterialien bzw. zur Herstellung von Adsorptionsfiltermaterialien verwendet werden. Derartige Adsorptionsfiltermaterialien lassen sich insbesondere für die Herstellung von Schutzbekleidung einsetzen, z. B. von Schutzanzügen, Schutzhandschuhen, Schutzunterwäsche, Schutzschuhen etc., insbesondere für den zivilen oder militärischen Bereich (z. B. ABC-Schutz).

Des weiteren eignen sich die erfindungsgemäßen Hochleistungsadsorbentien auch zur Verwendung im Bereich der Medizin oder Pharmazie, insbesondere als Arzneimittel oder Arzneimittelbestandteil.

Schließlich können die erfindungsgemäßen Hochleistungsadsorbentien auch als Sorptionsspeicher für Gase und Flüssigkeiten dienen.

Aufgrund ihrer hohen Gesamtporosität bei hoher Meso- und Makroporosität und ebenso einer gewissen Mikroporosität und gleichzeitig exzellenter mechanischer Beständigkeit mit exzellenten adsorptiven Eigenschaften sind die erfindungsgemäßen Hochleistungsadsorbentien vergleichbaren Adsorbentien des Standes der Technik deutlich überlegen.

Weitere Ausgestaltungen, Abwandlungen und Variationen der vorliegenden Erfindung sind für den Fachmann beim Lesen der Beschreibung ohne weiteres erkennbar und realisierbar, ohne daß er dabei den Rahmen der vorliegenden Erfindung verläßt.

**Tabelle 1: Vergleich physikochemischer Parameter von zwei erfindungsgemäßen Hochleistungsadsorbentien auf der Basis kugelförmiger Aktivkohle einerseits und handelsüblicher Aktivkohle in Kugelform der Fa. Kureha andererseits**

| | Erfindungsgemäße Aktivkohle I | | Erfindungsgemäße Aktivkohle II | | Handelsübliche Aktivkohle der Fa. Kureha |
|---|---|---|---|---|---|
| | Nach erstem Aktivierungsschritt (Zwischenprodukt) | Nach zweitem Aktivierungsschritt (Endprodukt) | Nach erstem Aktivierungsschritt (Zwischenprodukt) | Nach zweitem Aktivierungsschritt (Endprodukt) | |
| Gesamtporenvolumen (Gurvich) (p/p₀ = 0,995) [cm³/g] ** | 0,6267 | 1,7890 | 0,7510 | 3,1590 | 0,5891 |
| Mittlerer Porendurchmesser [Å] | 18,08 | 42,05 | 19,05 | 62,75 | 17,89 |
| BET (Multipoint, MP) (p/p₀ = 0,05-0,1) (ASTM D6556-04) [m²/g] ** | 1.387 | 1.702 | 1.577 | 2.013 | 1.317 |
| Mikroporenvolumen (Carbon Black) [cm³/g] * | 0,5524 | 0,5082 | 0,6211 | 0,5311 | 0,5240 |
| Anteil der Mikroporen am Gesamtporenvolumen [%] * | 88,1 | 28,4 | 82,7 | 16,8 | 88,95 |
| Adsorbiertes Volumen N₂ (p/p₀ = 0,25) gewichtsbezogen [cm³/g] ** | 368 | 463 | 422 | 563 | 349 |
| Adsorbiertes Volumen N₂ (p/p₀ = 0,25) volumenbezogen [cm³/cm³]** | 233 | 138 | 227 | 101 | 206 |
| Adsorbiertes Volumen N₂ (p/p₀ = 0,995) gewichtsbezogen [cm³/g] ** | 404 | 1.154 | 484 | 2.037 | 380 |
| Adsorbiertes Volumen N₂ (p/p₀ = 0,995) volumenbezogen [cm³/cm³ ]** | 256 | 344 | 261 | 365 | 224 |
| Mikroporenoberfläche (Carbon Black) [cm²/g] * | 1.342 | 1.261 | 1.499 | 1.288 | 1.271 |
| Summe Meso- und Makroporenvolumen (= sog. äußeres Porenvolumen) (Carbon Black) [cm³/g] *** | 0,0743 | 1,2808 | 0,1299 | 2,6279 | 0,07 |
| Anteil der Meso- und Makroporen am Gesamtporenvolumen (= Anteil des sog. äußeren Porenvolumens in bezug auf Gesamtporenvolumen) [%]*** | 11,9 | 71,6 | 17,3 | 83,2 | 11,1 |
| Oberfläche der Meso- und Makroporen (= sog. äußere Porenoberfläche) (Carbon Black) [cm²/g] *** | 45 | 441 | 78 | 725 | 46 |
| Anteil der Porenoberfläche der Meso- und Makroporen in bezug auf BET-Oberfläche (= Anteil der sog. äußeren Porenoberfläche in bezug auf BET-Oberfläche) (MP) [%]*** | 3,2 | 25,9 | 4,9 | 36,0 | 3,5 |
| Adsorbat | N₂ | N₂ | N₂ | N₂ | N₂ |
| Butanadsorption (ASTM D5742-95/00) [%] | 30,9 | 42,4 | 35,4 | 52,9 | 29,2 |
| Iodzahl (ASTM D4607-94/99) [mg/g] | 1.413 | 1.588 | 1.490 | 1.750 | 1.343 |
| Methylenblauzahl (CEFIC) [ml] | 19,9 | 34,8 | 27,2 | 38,9 | < 10 |
| Melassezahl (PACS) [dimensionslos] | 96 | 1.020 | 142 | 1.174 | < 100 |
| Abriebfestigkeit (interne Methode) [%] | 98,22 | 90,38 | 99,2 | 90,04 | < 90 |

| | | | | | |
|---|---|---|---|---|---|
| *Mikroporen: Poren mit Porendurchmessern ≤ 20 Å ** p/p₀ = Partialdruck oder Partialdruckbereich ***Meso-und Makroporen: Sammelbezeichnung für alle Poren mit Porendurchmessern > 20 Å | | | | | |

## Patentansprüche

1. Hochleistungsadsorbentien auf der Basis von Aktivkohle in Form von diskreten Aktivkohlekörnern, vorzugsweise in Kugelform, wobei
• das Gesamtporenvolumen nach Gurvich der Hochleistungsadsorbentien mindestens 0,8 cm³/g beträgt,
• mindestens 70 % des Gesamtporenvolumens der Hochleistungsadsorbentien durch Poren mit Porendurchmessern von mehr als 20 Å gebildet sind,
• die Hochleistungsadsorbentien einen mittleren Porendurchmesser von mehr als 25 Å aufweisen,
• die Hochleistungsadsorbentien eine BET-Oberfläche von mindestens 1.250 m²/g aufweisen und
• die Hochleistungsadsorbentien eine Iodzahl von mindestens 1.350 mg/g aufweisen.

2. Hochleistungsadsorbentien auf der Basis von Alctivkohle nach Anspruch 1, **dadurch gekennzeichnet, daß** das Gesamtporenvolumen nach Gurvich der Hochleistungsadsorbentien mindestens 1,0 cm³/g, vorzugsweise mindestens 1,2 cm³/g, beträgt und/oder daß das Gesamtporenvolumen nach Gurvich der Hochleistungsadsorbentien Werte von bis zu 2,0 cm³/g, insbesondere bis zu 2,5 cm³/g, vorzugsweise bis zu 3,0 cm³/g, besonders bevorzugt bis zu 3,5 cm³/g, erreicht.

3. Hochleistungsadsorbentien auf der Basis von Aktivkohle nach Anspruch 1 oder 2, **dadurch gekennzeichnet, daß** das durch Poren mit Porendurchmessern von mehr als 20 Å gebildete Porenvolumen nach Carbon Black der Hochleistungsadsorbentien im Bereich von 0,4 bis 3,3 cm³/g, insbesondere 0,8 bis 3,2 cm³/g, bevorzugt 1,0 bis 3,1 cm³/g, besonders bevorzugt 1,2 bis 3,0 cm³/g, ganz besonders bevorzugt 1,2 bis 2,8 cm³/g, liegt.

4. Hochleistungsadsorbentien auf der Basis von Aktivkohle nach einem oder mehreren der vorangehenden Anspruche, **dadurch gekennzeichnet, daß** mindestens 75 %, ganz besonders bevorzugt mindestens 80 %, des Gesamtporenvolumens der Hochleistungsadsorbentien durch das Porenvolumen von Poren mit Porendurchmessern von mehr als 20 Å gebildet sind und/oder daß 70 % bis 95 %, vorzugsweise 70 % bis 90 %, besonders bevorzugt 70 bis 85 %, des Gesamtporenvolumens der Hochlexstungsadsorbentien durch das Porenvolumen von Poren mit Porendurchmessern von mehr als 20 Å gebildet sind.

5. Hochleistungsadsorbentien auf der Basis von Aktivkohle nach einem oder mehreren der vorangehenden Ansprüche, **dadurch gekennzeichnet, daß** der mittlere Porendurchmesser der Hochleistungsadsorbentien mindestens 30 Å, insbesondere mindestens 35 Å, bevorzugt mindestens 40 Å, beträgt und/oder daß der mittlere Porendurchmesser der Hochleistungsadsorbentien im Bereich von 25 bis 75 Å, insbesondere 30 bis 75 Å, vorzugsweise 35 bis 70 Å, besonders bevorzugt 40 bis 65 Å, liegt,

6. Hochleistungsadsorbentien auf der Basis von Aktivkohle nach einem oder mehreren der vorangehenden Ansprüche, **dadurch gekennzeichnet, daß** die BET-Oberfläche der Hochleistungsadsorbentien im Bereich von 1.250 m²/g bis 2.800m²/g, insbesondere 1.400 bis 2.500 m²/g, vorzugsweise 1.500 bis 2.300 m²/g, besonders bevorzugt 1.600 bis 2.100 m²/g, liegt.

7. Hochleistungsadsorbentien auf der Basis von Aktivkohle nach einem oder mehreren der vorangehenden Ansprüche, **dadurch gekennzeichnet, daß** die Hochleistungsadsorbentien eine Butanadsorption von mindestens 30 %, insbesondere mindestens 35 %, vorzugsweise mindestens 40 %, aufweisen und/oder daß die Hochleistungsadsorbentien eine Butanadsorption im Bereich von 30 bis 80 %, insbesondere 35 bis 75 %, vorzugsweise 40 bis 70 %, aufweisen.

8. Hochleistungsadsorbentien auf der Basis von Aktivkohle nach einem oder mehreren der vorangehenden Ansprüche, **dadurch gekennzeichnet, daß** die Hochleistungsadsorbentien eine Iodzahl im Bereich von 1.350 bis 2.100 mg/g, insbesondere 1.350 bis 2.000 mg/g, vorzugsweise 1.350 bis 1.900 mg/g, aufweisen.

9. Hochleistungsadsorbentien auf der Basis von Aktivkohle nach einem oder mehreren der vorangehenden Ansprüche, **dadurch gekennzeichnet, daß** die Hochleistungsadsorbentien einen Methylenblauwert von mindestens 15 ml, insbesondere mindestens 17 ml, vorzugsweise mindestens 19 ml, aufweisen und/oder daß die Hochleistungsadsorbentien einen Methylenblauwert im Bereich von 15 bis 60 ml, insbesondere 17 bis 50 ml, vorzugsweise 19 bis 45 ml, aufweisen.

10. Hochleistungsadsorbentien auf der Basis von Aktivkohle nach einem oder mehreren der vorangehenden Ansprüche, **dadurch gekennzeichnet, daß** die Hochleistungsadsorbentien eine Melassezahl von mindestens 300, insbesondere mindestens 350, vorzugsweise mindestens 400, aufweisen und/oder daß die Hochleistungsadsorbentien eine Melassezahl im Bereich von 300 bis 1.400, insbesondere 350 bis 1.300, vorzugsweise 400 bis 1.250, ganz besonders bevorzugt 700 bis 1.200, aufweisen.

11. Verfahren zur Herstellung der Hochleistungsadsorbentien auf der Basis von Aktivkohle nach einem oder mehreren der vorangehenden Ansprüche, wobei ein kohlenstoffhaltiges Ausgangsmaterial in Form von sulfonierten StyrollDivinylbenzol-Copolymeren zunächst carbonisiert und nachfolgend aktiviert wird,
**dadurch gekennzeichnet,**
**daß** die Aktivierung zweistufig durchgeführt wird, wobei das carbonisierte Ausgangsmaterial zunächst in einem ersten Aktivierungsschritt einer Aktivierung in einer wasserdampfhaltigen Atmosphäre unterzogen wird, gefolgt von einem zweiten Aktivierungsschritt der Aktivierung in einer CO₂-haltigen Atmosphäre.

12. Verfahren nach Anspruch 11, **dadurch gekennzeichnet, daß** die Atmosphäre des ersten Aktivierungsschrittes Wasserdampf, insbesondere ein Gemisch aus Wasserdampf/Inertgas, vorzugsweise ein Gemisch aus Wasserdampf/Stickstoff, um faßt oder hieraus besteht, insbesondere wobei der Durchsatz an Wasserdampf 25 bis 350 m³/h, insbesondere 50 bis 300 m³/h, beträgt, bezogen auf reinen Wasserdampf, und/oder insbesondere wobei der massenbezogene Durchsatz an Wasserdampf 0,01 bis 50 l/(h · kg), insbesondere 0,02 bis 25 l/(h · kg), vorzugsweise 0,02 bis 5 l/(h · kg), beträgt, berechnet als Wasser und bezogen auf die Menge an mit Wasserdampf zu aktivierendem Ausgangsmaterial.

13. Verfahren nach einem oder mehreren der vorangehenden Ansprüche, **dadurch gekennzeichnet, daß** der erste Aktivierungsschritt bis zur Erreichung einer vorgegebenen Iodzahl, insbesondere bis zur Erreichung einer Iodzahl von mindestens 1.004 mg/g, insbesondere mindestens 1.250 mg/g, durchgeführt wird.

14. Verwendung der Hochleistungsadsorbentien auf der Basis von Aktivkohle nach einem oder mehreren der vorangehenden Ansprüche für die Adsorption von Giftstoffen, Schadstoffen und Gerüchen, insbesondere aus Gas- oder Luftströmen, oder zur Reinigung oder Aufbereitung von Gasen, insbesondere Luft oder Flüssigkeiten, insbesondere Wasser.

15. Verwendung der Hochleistungsadsorbentien auf der Basis von Aktivkohle nach einem oder mehreren der vorangehenden Ansprüche zur Verwendung in Adsorptionsfiltermaterialien, insbesondere für die Herstellung von Schutzbekleidung.

## Claims

1. High-performance adsorbents based on activated carbon in the form of discrete activated carbon grains, preferably of spherical shape, wherein
• the total pore volume of the high-performance adsorbents, according to the Gurvich rule, is at least 0.8 cm³/g,
• at least 70% of the total pore volume of the high-performance adsorbents is formed by pores having pore diameters of more than 20 Å,
• the high-performance adsorbents have a mean pore diameter of more than 25 Å,
• the high-performance adsorbents have a BET surface area of at least 1250 m²/g, and
• the high-performance adsorbents have an iodine number of at least 1350 mg/g.

2. High-performance adsorbents based on activated carbon according to claim 1, **characterized in that** the total pore volume of the high-performance adsorbents, according to the Gurvich rule, is at least 1.0 cm³/g, preferably at least 1.2 cm³/g, and/or **in that** the total pore volume of the high-performance adsorbents, according to the Gurvich rule, reaches values of up to 2.0 cm³/g, in particular up to 2.5 cm³/g, preferably up to 3.0 cm³/g, particularly preferably up to 3.5 cm³/g.

3. High-performance adsorbents based on activated carbon according to claim 1 or 2, **characterized in that** the pore volume, according to the carbon black method, of the high-performance adsorbents which is formed by pores having pore diameters of more than 20 Å lies in the range from 0.4 to 3.3 cm³/g, in particular 0.8 to 3.2 cm³/g, preferably 1.0 to 3.1 cm³/g, particularly preferably 1.2 to 3.0 cm³/g, more particularly preferably 1.2 to 2.8 cm³/g.

4. High-performance adsorbents based on activated carbon according to one or more of the preceding claims, **characterized in that** at least 75%, more particularly preferably at least 80%, of the total pore volume of the high-performance adsorbents is formed by the pore volume of pores having pore diameters of more than 20 Å, and/or **in that** 70% to 95%, preferably 70% to 90%, particularly preferably 70% to 85%, of the total pore volume of the high-performance adsorbents is formed by the pore volume of pores having pore diameters of more than 20 Å.

5. High-performance adsorbents based on activated carbon according to one or more of the preceding claims, **characterized in that** the mean pore diameter of the high-performance adsorbents is at least 30 Å, in particular at least 35 Å, preferably at least 40 Å, and/or **in that** the mean pore diameter of the high-performance adsorbents lies in the range from 25 to 75 Å, in particular 30 to 75 Å, preferably 35 to 70 Å, particularly preferably 40 to 65 Å.

6. High-performance adsorbents based on activated carbon according to one or more of the preceding claims, **characterized in that** the BET surface area of the high-performance adsorbents lies in the range from 1250 m²/g to 2800 m²/g, in particular 1400 to 2500 m²/g, preferably 1500 to 2300 m²/g, particularly preferably 1600 to 2100 m²/g.

7. High-performance adsorbents based on activated carbon according to one or more of the preceding claims, **characterized in that** the high-performance adsorbents have a butane adsorption of at least 30%, in particular at least 35%, preferably at least 40%, and/or **in that** the high-performance adsorbents have a butane adsorption in the range from 30 to 80%, in particular 35 to 75%, preferably 40 to 70%.

8. High-performance adsorbents based on activated carbon according to one or more of the preceding claims, **characterized in that** the high-performance adsorbents have an iodine number in the range from 1350 to 2100 mg/g, in particular 1350 to 2000 mg/g, preferably 1350 to 1900 mg/g.

9. High-performance adsorbents based on activated carbon according to one or more of the preceding claims, **characterized in that** the high-performance adsorbents have a methylene blue value of at least 15 ml, in particular at least 17 ml, preferably at least 19 ml, and/or **in that** the high-performance adsorbents have a methylene blue value in the range from 15 to 60 ml, in particular 17 to 50 ml, preferably 19 to 45 ml.

10. High-performance adsorbents based on activated carbon according to one or more of the preceding claims, **characterized in that** the high-performance adsorbents have a molasses number of at least 300, in particular at least 350, preferably at least 400, and/or **in that** the high-performance adsorbents have a molasses number in the range from 300 to 1400, in particular 350 to 1300, preferably 400 to 1250, more particularly preferably 700 to 1200.

11. Method for producing the high-performance adsorbents based on activated carbon according to one or more of the preceding claims, wherein a carbonaceous starting material in the form of sulphonated styrene-divinylbenzene copolymers is first carbonized and then activated, **characterized in that** the activation is carried out in two stages, wherein the carbonized starting material is first subjected, in a first activation step, to an activation in an atmosphere containing water vapour, followed by a second activation step of activation in an atmosphere containing CO₂.

12. Method according to claim 11, **characterized in that** the atmosphere of the first activation step comprises or consists of water vapour, in particular a mixture of water vapour/inert gas, preferably a mixture of water vapour/nitrogen, in particular wherein the throughput of water vapour is 25 to 350 m³/h, in particular 50 to 300 m³/h, based on pure water vapour, and/or in particular wherein the mass-based throughput of water vapour is 0.01 to 50 l/(h · kg), in particular 0.02 to 25 l/(h · kg), preferably 0.02 to 5 l/(h · kg), calculated as water and based on the quantity of starting material to be activated with water vapour.

13. Method according to one or more of the preceding claims, **characterized in that** the first activation step is carried out until a predefined iodine number is achieved, in particular until an iodine number of at least 1000 mg/g, in particular at least 1250 mg/g, is achieved.

14. Use of the high-performance adsorbents based on activated carbon according to one or more of the preceding claims for the adsorption of toxic substances, noxious substances and odours, in particular from gas streams or air streams, or for cleaning or purifying gases, in particular air, or liquids, in particular water.

15. Use of the high-performance adsorbents based on activated carbon according to one or more of the preceding claims for use in adsorptive filtering materials, in particular in the manufacture of protective clothing.

## Revendications

1. Adsorbants hautes performances à base de charbon actif sous forme de grains discrets de charbon actif, de préférence sous forme sphérique, dans lesquels
- le volume total des pores selon Gurvich des adsorbants hautes performances est d'au moins 0,8 cm³/g,
- au moins 70 % du volume total des pores des adsorbants hautes performances sont formés de pores ayant un diamètre des pores supérieur à 20 Å,
- les adsorbants hautes performances présentent un diamètre moyen des pores supérieur à 25 Å,
- les adsorbants hautes performances présentent une aire BET d'au moins 1250 m²/g, et
- les adsorbants hautes performances présentent un indice d'iode d'au moins 1350 mg/g.

2. Adsorbants hautes performances à base de charbon actif selon la revendication 1, **caractérisés en ce que** le volume total des pores selon Gurvich des adsorbants hautes performances est d'au moins 1,0 cm³/g, de préférence d'au moins 1,2 cm³/g, et/ou que le volume total des pores selon Gurvich des adsorbants hautes performances atteint des valeurs allant jusqu'à 2,0 cm³/g, en particulier jusqu'à 2,5 cm³/g, de préférence jusqu'à 3,0 cm³/g, d'une manière particulièrement préférée jusqu'à 3,5 cm³/g_{.}

3. Adsorbants hautes performances à base de charbon actif selon la revendication 1 ou 2, **caractérisés en ce que** le volume des pores formé par des pores ayant un diamètre des pores supérieur à 20 Å par la méthode au noir de carbone des adsorbants hautes performances est compris dans la plage de 0,4 à 3,3 cm³/g, en particulier de 0,8 à 3,2 cm³/g, de préférence de 1,0 à 3,1 cm³/g, d'une manière particulièrement préférée de 1,2 à 3,0 cm³/g, d'une manière tout particulièrement préférée de 1,2 à 2,8 cm³/g.

4. Adsorbants hautes performances à base de charbon actif selon l'une ou plusieurs des revendications précédentes, **caractérisés en ce qu'**au moins 75 %, d'une manière tout particulièrement préférée au moins 80 % du volume total des pores des adsorbants hautes performances sont formés par le volume de pores des pores ayant un diamètre des pores supérieur à 20 Å, et/ou que 70 % à 95 %, de préférence 70 à 90 %, d'une manière particulièrement préférée 70 à 85 % du volume total des pores des adsorbants hautes performances sont formés par le volume de pores des pores ayant un diamètre des pores supérieur à 20 Å.

5. Adsorbants hautes performances à base de charbon actif selon l'une ou plusieurs des revendications précédentes, **caractérisés en ce que** le diamètre moyen des pores des adsorbants hautes performances est d'au moins 30 Å, en particulier d'au moins 35 Å, de préférence d'au moins 40 Å, et/ou que le diamètre moyen des pores des adsorbants hautes performances est compris dans la plage de 25 à 75 Å, en particulier de 30 à 75 Å, de préférence de 35 à 70 Å, d'une manière particulièrement préférée de 40 à 65 Å.

6. Adsorbants hautes performances à base de charbon actif selon l'une ou plusieurs des revendications précédentes, **caractérisés en ce que** l'aire BET des adsorbants hautes performances est comprise dans la plage de 1250 m²/g à 2800 m²/g, en particulier de 1400 à 2500 m²/g, de préférence de 1500 à 2300 m²/g, d'une manière particulièrement préférée de 1600 à 2100 m²/g.

7. Adsorbants hautes performances à base de charbon actif selon l'une ou plusieurs des revendications précédentes, **caractérisés en ce que** les adsorbants hautes performances présentent une adsorption du butane d'au moins 30 %, en particulier d'au moins 35 %, de préférence d'au moins 40 %, et/ou que les adsorbants hautes performances présentent une adsorption du butane comprise dans la plage de 30 à 80 %, en particulier de 35 à 75 %, de préférence de 40 à 70 %.

8. Adsorbants hautes performances à base de charbon actif selon l'une ou plusieurs des revendications précédentes, **caractérisés en ce que** les adsorbants hautes performances présentent un indice d'iode compris dans la plage de 1350 à 2100 mg/g, en particulier de 1350 à 2000 mg/g, de préférence de 1350 à 1900 mg/g.

9. Adsorbants hautes performances à base de charbon actif selon plusieurs des revendications précédentes, **caractérisés en ce que** les adsorbants hautes performances présentent un indice de bleu de méthylène d'au moins 15 ml, en particulier d'au moins 17 ml, de préférence d'au moins 19 ml, et/ou que les adsorbants hautes performances présentent un indice de bleu de méthylène compris dans la plage de 15 à 60 ml, en particulier de 17 à 50 ml, de préférence de 19 à 45 ml.

10. Adsorbants hautes performances à base de charbon actif selon l'une ou plusieurs des revendications précédentes, **caractérisés en ce que** les adsorbants hautes performances présentent un indice de mélasse d'au moins 300, en particulier d'au moins 350, de préférence d'au moins 400, et/ou que les adsorbants hautes performances présentent un indice de mélasse compris dans la plage de 300 à 1400, en particulier de 350 à 1300, de préférence de 400 à 1250, d'une manière tout particulièrement préférée de 700 à 1200.

11. Procédé de fabrication des adsorbants hautes performances à base de charbon actif selon l'une ou plusieurs des revendications précédentes, dans lequel une matière de départ contenant du carbone est d'abord carbonisée sous forme de copolymères styrène sulfoné/divinylbenzène, puis est activée, **caractérisé en ce que** l'activation est mise en oeuvre en deux étapes, la matière de départ carbonisée étant d'abord, dans une première étape d'activation, soumise à une activation dans une atmosphère contenant de la vapeur d'eau, puis, dans une deuxième étape d'activation, à l'activation dans une atmosphère contenant du CO₂.

12. Procédé selon la revendication 11, **caractérisé en ce que** l'atmosphère de la première étape d'activation comprend de la vapeur d'eau, en particulier un mélange de vapeur d'eau et d'un gaz inerte, de préférence un mélange de vapeur d'eau et d'azote, ou en est constituée, en particulier dans lequel le débit de la vapeur d'eau est de 25 à 350 m³/h, en particulier de 50 à 300 m³/h, rapporté à la vapeur d'eau pure, et/ou en particulier dans lequel le débit massique de la vapeur d'eau est de 0,01 à 50 l/(h.kg), en particulier de 0,02 à 25 l/(h.kg), de préférence de 0,02 à 5 l/(h.kg), calculé en eau et rapporté à la quantité de matière de départ devant être activée par de la vapeur d'eau.

13. Procédé selon l'une ou plusieurs des revendications précédentes, **caractérisé en ce que** la première étape d'activation est mise en oeuvre jusqu'à ce que l'on atteigne un indice d'iode prédéfini, en particulier jusqu'à ce que l'on atteigne un indice d'iode d'au moins 1000 mg/g, en particulier d'au moins 1250 mg/g.

14. Utilisation des adsorbants hautes performances à base de charbon actif selon l'une ou plusieurs des revendications précédentes pour l'adsorption de poisons, de polluants et d'odeurs, provenant en particulier de courants de gaz ou d'air, ou pour la purification ou le traitement de gaz, en particulier l'air, ou de liquides, en particulier l'eau.

15. Utilisation des adsorbants hautes performances à base de charbon actif selon l'une ou plusieurs des revendications précédentes pour utilisation dans des matériaux filtrants par adsorption, en particulier pour la fabrication d'un vêtement de protection.
